Europäisches Patentamt

European Patent Office

Office européen des brevets

(11) Publication number: **0 445 681 A2**

# EUROPEAN PATENT APPLICATION

(21) Application number: 91103184.7

(22) Date of filing: 04.03.91

(51) Int. Cl.⁵: **C12N 15/12**, C12N 1/21, C12P 21/02

(30) Priority: 06.03.90 JP 54446/90

(43) Date of publication of application:
11.09.91 Bulletin 91/37

(84) Designated Contracting States:
BE CH DE ES FR GB IT LI NL SE

(71) Applicant: KOWA COMPANY, LTD.
6-29, 3-chome Nishiki
Naka-ku Nagoya-shi Aichi-ken(JP)

(72) Inventor: Kimura, Shigeru
3-59-10, Minamimachi
Higashi-Yamato-shi, Tokyo(JP)
Inventor: Mizoguchi, Toshimi
1314-3-703, Fujisawa
Iruma-shi, Saitama(JP)
Inventor: Okuchi, Masao
3-9-5, Nakaarai
Tokorozawa-shi, Saitama(JP)
Inventor: Doi, Takeshi
1-28-6, Minamicho, Hanakoganei
Kodaira-shi, Tokyo(JP)
Inventor: Iwasaki, Akio
1-14-1-302, Tokiwadaira
Matsudo-shi, Chiba(JP)

(74) Representative: Wächtershäuser, Günter, Dr.
Tal 29
W-8000 München 2(DE)

(54) **Preparation of a thrombin-binding substance.**

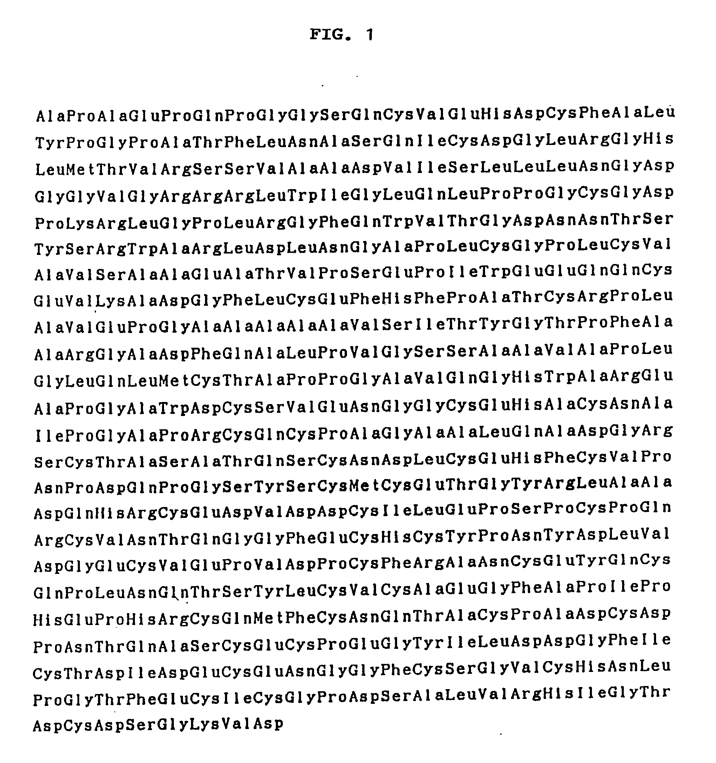

(57) A DNA fragment containing a nucleotide sequence capable of encoding the amino acid sequence as shown in Fig. 1, and a process for preparing a thrombin-binding substance according to a genetic engineering technique are disclosed.

EP 0 445 681 A2

## BACKGROUND OF THE INVENTION

1) Field of the Invention:

This invention relates to a DNA fragment encoding a thrombin-binding substance, a recombinant plasmid containing the DNA fragment, a transformant containing the recombinant plasmid, and a preparation of the thrombin-binding substance.

2) Description of the Background Art:

Heretofore, many researches have been conducted on the roles played by a protease thrombin in the blood coagulation system, and by now, mechanism of blood coagulation has almost been elucidated. Recently, it has been reported that thrombin activates protein C in the living body, and that a factor acting as a coenzyme on this mechanism was found in a tissue extract of a rabbit lung, which was named thrombomodulin (N.L. Esmon et al., J. Biol. Chem., 257, (2) 859-864 (1982)). Here, Protein C is said to participate in the fibrinolysis and anticoagulant systems.

Aoki et al. isolated a human thrombomodulin having the similar properties from the human placenta and reported that it had a molecular weight of about 71,000 (non-reduced state) (Thrombo. Res. 37, 353-364 (1985)).

I. Maruyama et al. compared the activities of a human thrombomodulin isolated from the human placenta and having a molecular weight of about 75,000 and the mentioned rabbit thrombomodulin, and reported that they had the identical activities (J. Clin. Invest., 75, 987-991 (1985)).

H. Ishii et al. reported that substances having identical activities as the thrombomodulin were present in human plasma and in human urine, and their molecular weights were about 63,000 and 54,000 (J. Clin. Invest., 76, 2178-2181 (1985)).

The present inventors previously found, in human urine, two thrombin-binding substances which were different from any of the above substances and had smaller molecular weights (MW about 39,000 and 31,000 in a non-reduced state), and applied a patent (Japanese patent application laid-open (Kokai) No. 146898/1988).

The present inventors further separated the following two thrombin-binding substances (A) and (B) which were different from the above substances from a human tissue culture and from human urine, and filed patent applications (Japanese Patent Application Nos. 214139/1988, 116471/1990 and 202027/1990). These thrombin-binding substances (A) and (B) have the following properties and useful as a fibrinolysis accelerator or an anticoagulant drug.

    (a) Molecular Weight:
        (A)     90,000 - 92,000 in a reduced state, and
                 55,000 - 58,000 in a non-reduced state,
        (B)     98,000 - 105,000 in a reduced state, and
                 60,000 - 65,000 in a non-reduced state,
    (b) Isoelectric point:
        (A)     pH 6.0 - 6.8,
        (B)     pH 5.8 - 6.5,
    (c) Affinity:
    Both (A) and (B) have an intense affinity to thrombin,
    (d) Activity:
       1) Both (A) and (B) bind to thrombin for the activation of protein C.
       2) Both (A) and (B) prolong the clotting time.
    (e) Stability:
    Both (A) and (B) are stable against denaturators (sodium dodecyl sulfate, urea).

However, no reports have ever been made concerning the gene of the substances (A) and (B) or the primary structure of the amino acid sequence of the protein which constitutes these substances. In other words, preparation of the thrombin-binding substances (A) and (B) by the genetic engineering technique has not yet been reported before the present invention.

Under the above-mentioned circumstances, the present inventors have made extensive studies and have found that a DNA fragment encoding thrombin-binding substances (A) and (B) is obtainable from the human placental genomic DNA by using synthesized DNA primers as a probe, and that thrombin-binding substances (A) and (B) (hereinafter may be referred to simply as thrombin-binding substances) can be prepared by transforming the host cells by the use of a recombinant plasmid containing the DNA fragment thereby expressing the thrombin-binding substance gene. This invention was accomplished based on these findings.

SUMMARY OF THE INVENTION

An object of this invention is to provide a DNA fragment having a nucleotide sequence capable of encoding the amino acid sequence shown in Fig. 1.

Another object of the invention is to provide a replicative recombinant plasmid containing the DNA fragment and a replicative expression vector.

A further object of the invention is to provide a transformant cell containing the recombinant DNA.

A further object of the invention is to provide a process for preparing the thrombin-binding substances.

BRIEF DESCRIPTION OF THE DRAWINGS

Fig. 1 shows the amino acid sequence of the thrombin-binding substances (A) and (B) prepared according to the process of this invention;

Fig. 2 shows a nucleotide sequence of the DNA fragment of the present invention;

Fig. 3 shows a nucleotide sequence of a 1.65 kb fragment inserted into the expression vector pCDM-UTM1 of the present invention; and

Fig. 4 shows a process of constructing the expression vector pCDM-UTM1 of the present invention.

DETAILED DESCRIPTION OF THE INVENTION AND PREFERRED EMBODIMENTS

The DNA fragment encoding the thrombin-binding substances of the present invention can be prepared by the following process:

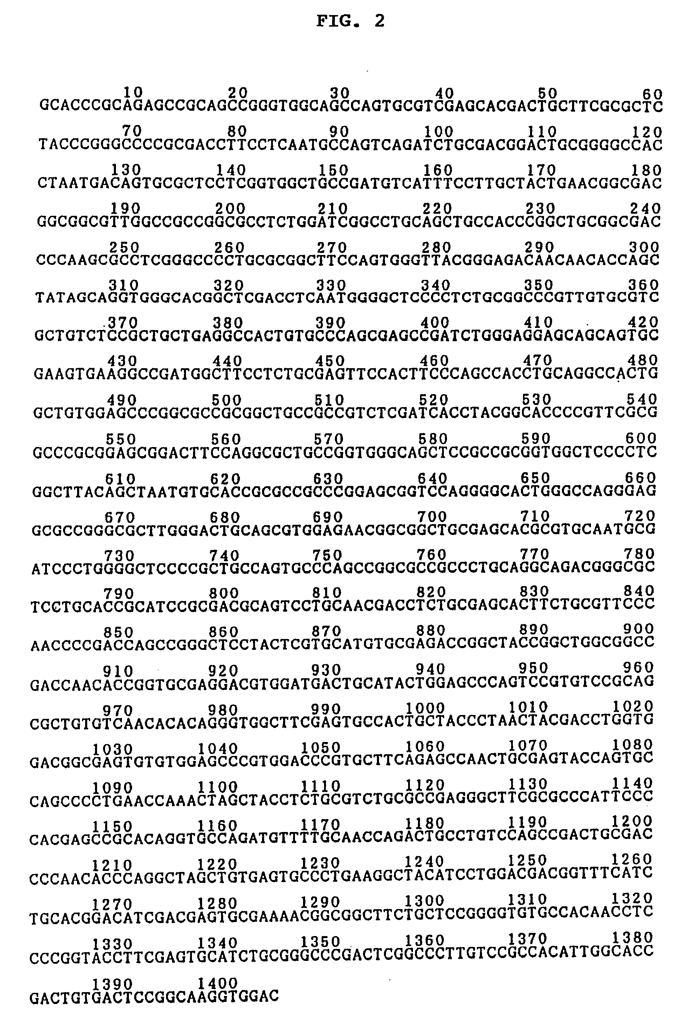

First, a human placental genomic DNA is cleaved with a suitable restriction endonuclease to make a template DNA. Then DNA primers are synthesized with reference to a known nucleotide sequence of a human thrombomodulin gene (Shirai, T. et al: J. Biochem. 103, 281-285(1988)). These primers serve as a probe, and the template DNA is subjected to a screening step. The obtained DNA is cleaved with a suitable restriction endonuclease and a produced DNA fragment is ligated with a cloning vector for transformation of the microorganism. Plasmid DNA is extracted from the obtained transformant and after the treatment with a restriction endonuclease, a DNA fragment containing the DNA fragment of this invention can be obtained. The nucleotide sequence of the DNA fragment of this invention is shown in Fig. 2. It should however be noted that the nucleotide sequence is not necessarily limited to the sequence of Fig. 2 so far as it is capable of encoding the amino acid sequence constituting the peptide of the target thrombin-binding substances.

In order to construct a recombinant plasmid containing the DNA fragment of this invention, a replicative expression vector is bonded to the DNA fragment of this invention.

Examples of the usable expression vectors include any vectors such as those derived from procaryotes typified by E. coli, derived from yeasts, insect viruses, and from vertebrate viruses.

In order to effectively produce the thrombin-binding substances, it is preferred to construct a recombinant vector for expression which contains the following nucleotide sequences (1) to (7) in order in the downstream direction of transcription:

(1) a nucleotide sequence acting as a promoter,

(2) a nucleotide sequence serving as a ribosome-binding site,

(3) a nucleotide sequence serving as an initiation codon,

(4) a nucleotide sequence capable of coding a signal peptide,

(5) the DNA fragment of this invention,

(6) a nucleotide sequence serving as a termination codon, and

(7) a nucleotide sequence acting as a polyA signal.

DNA used as a vector is preferably plasmid. An example of such plasmid is one capable of being amplified in host cells of E. coli, and also capable of expressing the inserted gene by the transformation of mammalian cells. The plasmid DNA has a DNA sequence required for the multiplication of the plasmid in cells of E. coli, for example, a DNA sequence of the starting region of replication of Co1E1 plasmid, another DNA sequence capable of serving as a promoter in the mammalian cells, a gene capable of acting as a selective marker in a transformant of E. coli and a gene capable of acting as a selective marker in a transformant of the mammalian cells. More preferably, the plasmid DNA has a DNA sequence of the starting region of replication such as HSV ori, polyoma ori and SV40 ori capable of acting in mammalian cells. Illustrative examples of the promoter may include promoters of cytomegalo virus, SV40, polyoma virus, bovine papilloma virus and adeno virus; LTR of retrovirus such as MMTV; and a promoter of metallothionein gene. As selective marker genes, may be mentioned ampicillin-resistant genes, kanamycin-resistant genes, tetracycline-resistant genes, chloramphenicol-resistant genes and so on. As selective marker genes of mammalian cells, may be mentioned neomycin-resistant genes, hygromycin B-resistant genes, thymidine kinase genes, Eco gpt and dihydrofolate reductase genes. These genes may be used either singly or in combination.

The incorporation of a DNA fragment of this invention in the mentioned vectors is effected by cleaving the DNA having such fragment with a suitable restriction endonuclease, and after adding a suitable linker if needed, joining the resultant DNA fragment to a vector DNA which has been cleaved with a suitable restriction endonuclease. Examples of the restriction endonuclease include EcoR I, Sph I, Pst I, Hind II, Hind III, BamH I, Xho I, Xba I, Ban III, Sma I and Nco I. Nuceotide modifying enzymes can also be used, which include exonuclease III, Bal 31, S1 nuclease, exonuclease VII, mung bean nuclease and DNA polymerase I. Usable linkers include EcoR I linker, Sma I linker, Nco I linker, BamH I linker, Xho I linker, Hind III linker, Pst I linker, Sph I linker and Xba I linker.

Introduction of the resultant plasmid for expression into host cells by the competent cell method, protoplasts method, calcium phosphate coprecipitation method, electroporation method, DEAE dextran method, lipofectin method or the like permits production of a transformant which has ability of effectively producing the recombinant plasmid and/or the thrombin-binding substances of this invention. Illustrative host cells for obtaining the mentioned transformants are unicellular microorganisms such as bacteria and yeasts, cultured insect cells and cultured vertebrate cells. When E. coli is chosen as a host, various variants of the K12 strain of E. coli, for example, HB101, C600K, JM101, JM103, JM105, JM109, MV1034, MV1184, MC1061/P3 and the like may be used. When cultured mammalian cells are chosen as a host, COS cells, CHO cells, L cells, C127 cells, NIH3T3 cells, HeLa cells and the like may be used.

A thrombin-binding substance is prepared by culturing the obtained transformant and then extracting and isolating the same from the culture medium and/or the cultured cells.

Upon culture of the transformant, various natural and synthetic culture media are used. They may desirably contain carbon sources such as sugars, alcohols or organic acid salts; nitrogen sources such as protein mixtures, amino acids or ammonium salts; and inorganic salts. It is also desired to add vitamins and antibiotics corresponding to the associated selective marker genes. If the plasmid allows to control the expression, it is necessary to follow a procedure which will induce the expression in the course of culturing. After culturing, culture medium and cultured cells are centrifugally separated. Where the thrombin-binding substances accumulate in the cultured cells, it is necessary to disrupt or fracture the cells by freezing and thawing, ultrasonic processing, French press, enzyme treatment, homogenizing or the like, and then to solubilize the thrombin-binding substances with EDTA, surfactants, urea, guanidine hydrochloride or the like.

The resultant culture medium or cultured cell extract containing the thrombin-binding substances is subjected to chromatography on one of various columns, so that purified thrombin-binding substances are obtained. As column chromatography, ion-exchange chromatography, affinity chromatography (for example, monoclonal antibody described in Japanese patent application laid-open (Kokai) No. 45398/1989 is usable) and gel chromatography may be applied either singly or in combination.

The thus-obtained thrombin-binding substances have the aforementioned properties, and have the amino acid sequence shown in Table 1. They may have one or more sugar residues. When yeasts or vertebrate cells are used as host cells, glycosylated peptides are generally obtained.

As described above, the gene of high molecular thrombin-binding substances (A) and (B) which are useful as an anticoagulant drug or a fibrinolysis accelerator and having a calcium-binding site has now been elucidated by the present inventors. Furthermore, economical mass production of the thrombin-binding substances is achieved by this invention.

Examples

This invention will now be explained by way of examples, which should not be construed as limiting the invention thereto.

Example 1:

Cloning of thrombin-binding substance gene:

The nucleotide sequence of a human thrombomodulin gene was referred to (Shirai, T. et al., J. Biochem. 103, 281-285, 1988), and the following DNA primers were synthesized using a DNA synthesizer (ABI model 381A):

Primer #1       5'-AGGGCCGGGCACTTATAAACT-3'

Primer #2       5'-CCCAGTGGTCCAGTGACGTCA-3'

In the next place, human placental genome DNA (by Clontech Laboratories) was cleaved with BamH I to obtain a template DNA for amplification of the gene: Quick Thermo System (Model QTS-10M, by Nippon Genetics K.K.) was used, and thirty cycles of an amplification process of 94°C for 2 minutes, 50°C for 3 minutes and 72°C for 4 minutes were effected. After completion of the reaction, a portion of the reaction mixture was taken and agarose gel electrophoresis confirmed that the DNA band having a target size was amplified. The reaction mixture consisted of:

| | |
|---|---|
| Distilled water | 71 microliters |
| 10 X buffer | 10 " |
| dNTP mixture sol. (2.5 mM) | 8 " |
| Primer #1 (20 micro M) | 5 " |
| Primer #2 (20 micro M) | 5 microliters |
| Template DNA (micro g/microliter) | 1 " |
| AmpliTaq (5 units/micro liter) | 0.5 " |

10 X buffer:

0.1 M potassium chloride

0.1 M Tris-HCl (pH8.3)

0.1% gelatin

15 mM magnesium chloride

DNAs were collected from the reaction mixture by ethanol precipitation, cleaved with Xho I and Kpn I, and a 1.57 kb Kho I - Kpn I fragment was prepared by agarose gel electrophoresis. Separately, a vector pUC118 for cloning (Vieira, J. and Messing, J.: Methods Enzymol. 153, 3-11, 1987) was cleaved with Hind II, joined to Xho I linker and then cleaved with Xho I and Kpn I to prepare a vector fragment by agarose electrophoresis. The above-described vector fragment, 1.57 kb Xho I - Kpn I fragment were ligated and E. coli MV 1304 (Vieira, J. and Messing, J.: Methods Enzymol. 153, 3-11, 1987) was transformed.

Plasmid DNA was extracted from the obtained transformant and cleaved with a restriction endonuclease. As a result, 6 clones containing a plasmid inserted with 1.57 kb Xho I - Kpn I fragment derived from a human thrombomodulin gene were selected. In the process of determining the nucleotide sequence of the inserted fragment of the 6 clones, it was revealed that all of them had one to three mutations. A 0.31 kb Xho I - Sma I fragment of clone 2, a 0.65 kb Sma I - Mlu I fragment of clone 1 and a 0.62 kb Mlu I - Kpn I fragment of clone 4, which had no mutations, were ligated with the mentioned vector fragment again for constructing a plasmid pUCTM/XHO-KPN containing a fragment having a correct nucleotide sequence of this invention inserted thereto. The nucleotide sequence of the DNA fragment of this invention, which was inserted into the obtained plasmid pUCTM/XHO-KPN is shown in Fig. 2. The amino acid sequence corresponding to this nucleotide sequence is shown in Fig. 1, and this was confirmed to be identical to the sequence separately determined by the amino acid analysis of thrombin-binding substances (A) and (B).

Example 2:

Construction of a plasmid for expression of the thrombin-binding substances:

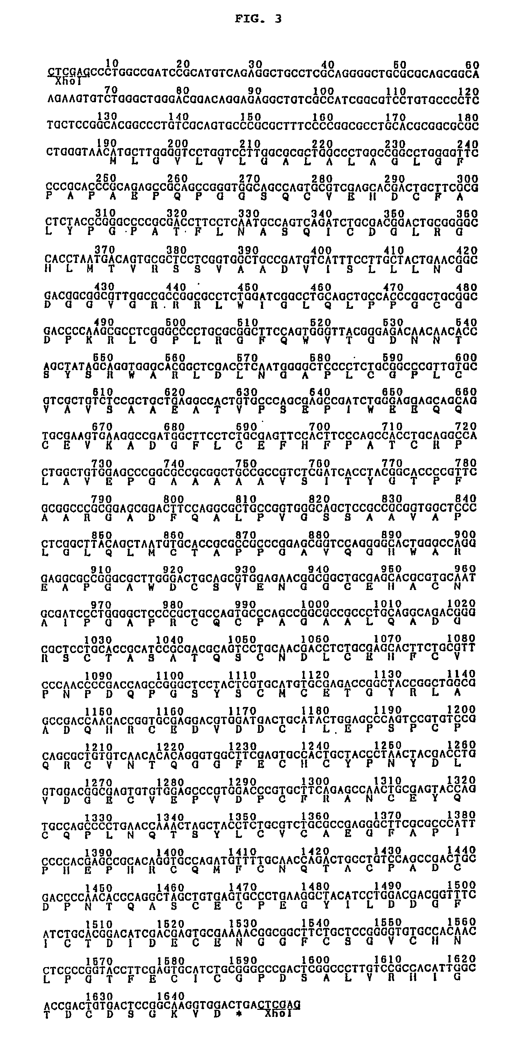

In order to express the amino acid sequence up to the C terminal Asp (486; Met = 1 in Fig. 3) determined by the amino acid analysis of thrombin-binding substances (A) and (B), the following linkers including the termination codon were synthesized and 5' terminal was phosphorylated.

Linker $1   5'-CTTCGAGTGCATCTGCGGGCCCGAC

                TCGGCCCTTGTC-3'

Linker $2   3'-CATGGAAGCTCACGTAGACGCCCGG

                GCTGAGCCGGGAACAGGCGGTG-5'

Linker $3   5'-CGCCACATTGGCACCGACTGTGACT

                CCGGCAAGGTGGACTGAC-3'

Linker $4   3'-TAACCGTGGCTGACACTGAGGCCGT

                TCCACCTGACTGAGCT-5'

A pUCTM/XHO-KPN was cleaved with Xho I and Kpn I, and was prepared a 1.57 kb Xho - Kpn I fragment derived from a human thrombomodulin gene by agarose gel electrophoresis. This 1.57 kb fragment, together with linkers $1, $2, $3 and $4, was ligated with an expression vector CDM 8 (manufactured by Imvitrogen Co.) which was dephosphorylated after the cleavage with Xho I, and E. coli MC1061/P3 (Seed, B. and Aruffo, A.: Proc. Natl. Acd. Sci. USA. 84, 3365-3369, 1987) was transformed. From the obtained transformant, a plasmid DNA was extracted and was subjected to a cleavage with a restriction endonuclease, and the direction of the insertion and the inserted fragment were investigated. From 4 clones which showed correct insertion direction and a correct restriction endonuclease map, 1.65 kb fragment containing the DNA fragment of this invention was cut out by the cleavage with Xho I to confirm the nucleotide sequence. As a result, all clones showed the nucleotide sequence of Fig. 3, which evidenced that the expression vector of this invention was correctly constructed.

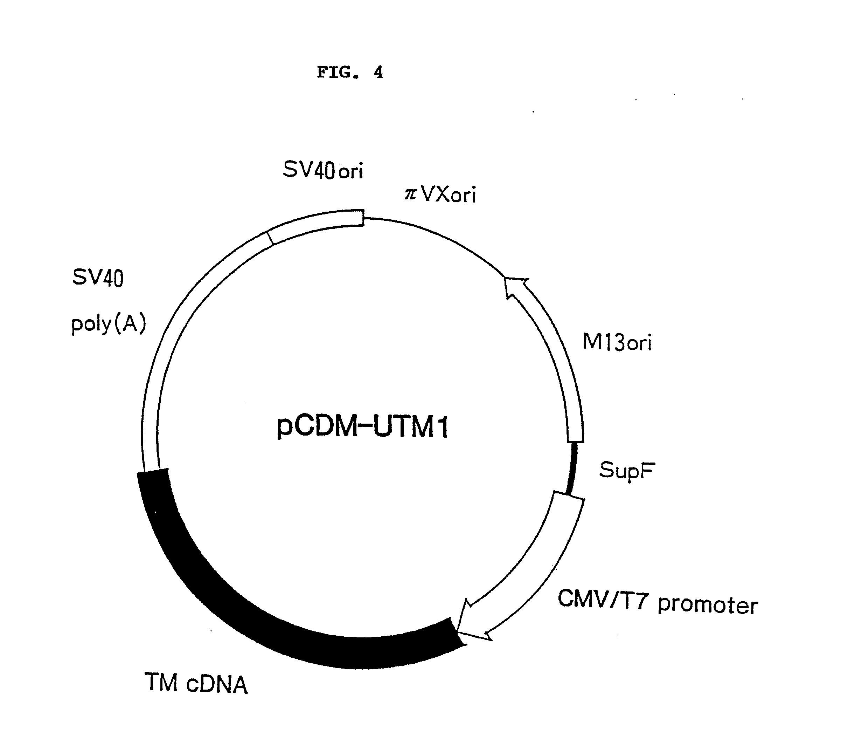

The expression vector of this invention thus constructed and the transformant containing this vector were named pCDM-UTM1 (Fig. 4) and E. coli MC1061/P3[pCDM-UTM1], respectively.

Example 3:

Expression of the thrombin-binding substances by the use of cultured animal cells:

According to an DEAE-Dextran method (Seed, B. and Aruffo, A.: Proc. Natl. Acad. Sci. USA. 84, 3365-3369, 1987), COS7 cells were transfected with pCDM-UTM1. Onto a 60 mm ⌀ culture dish was planted COS7 cells in the number of 1 X 10⁵. In the following day, the culture medium was suctioned and replaced with 2 ml of Dulbecco's modified minimum essential medium (DMEM) containing 10% Nu-serum (Collaborative Research Inc.). Into 100 microliters of a solution of DEAE-Dextran (average molecular weight: $5 \times 10^5$; by Pharmacia) which was dissolved in PBS to have a concentration of 10 mg/ml, 10 micrograms (1 microgram/microliter) of pCDM-UTM 1 was added. This was added to the cell culture broth along with 10 microliters of 20 mM chloroquine. Culture was continued for 4 hours at 37°C, followed by a suction of the culture medium, addition of 2 ml of 10% DMSO (dissolved in PBS), allowing to stand at room temperature for 2 minutes. After removing the DMSO solution by suction, 3 ml of DMEM containing 10% FCS was added thereto, followed by a culture for 48 hours at 37°C. The culture medium was changed to a serum free DMEM and additional culture was carried out for 48 hours before collecting a supernatant.

The obtained culture medium was passed through a column of 1 ml Sepharose 4B (2 mg IgG/ml resin) which was coupled with a monoclonal antibody A-73. The column was washed by; 1) 0.1 M sodium chloride-added 0.02 M Tris-HCl (pH 7.4): 2 ml, 2) 1 M sodium chloride and 0.05% Tween 20-added 0.02 M Tris-HCl (pH 7.4): 20 ml, and 3) 1 M sodium chloride-added 0.02 M Tris-HCl (pH 7.4): 5 ml in this order, and eluted with 5 ml of 0.02 M Tris-HCl (pH 7.4) added with 2 M of sodium thiocyanate, 5 mM EDTA and 1 M sodium chloride. The eluate was dialyzed against 0.02 M Tris-HCl added with 0.1 M sodium chloride. The dialysate was subjected to a SDS-PAGE according to a method proposed by Laemmli (Nature 227, 680-685). Proteins in the gel were transfered onto a PVDF membrane according to a method proposed by Matsudaira (J. Biol. Chem. 262 (21), 10035-10038). Next, the PVDF membrane was subjected to a reaction for 2 hours at room temperature in a 0.05 M Tris-HCl (TBS) containing 0.1% bovine serum albumin. Solution was decanted and washed throughtly with 0.05% Tween 20-added TBS, followed by a reaction for 1 hour at room temperature in a 0.05% Tween 20-added TBS containing a monoclonal antibody A-60 labeled with horseradish peroxidase. Solution was decanted and washed throughtly with 0.05% Tween 20-added TBS, followed by a color-developing in 5 ml of 50 mM acetic acid buffer (pH 5.0) containing 5 mg of 3-amino-9-ethylcarbazol dissolved in 2 ml of DMF and 25 microliters of 30% aqueous hydrogen peroxide. Single band was confirmed.

The thus obtained substance showed properties of thrombin-binding substances, including activation of protein C when combined with thrombin, and ability of prolonging the clotting time.

## Claims

1.  A DNA fragment containing a nucleotide sequence capable of encoding the amino acid sequence as shown in Fig. 1.

2.  A DNA fragment according to Claim 1, wherein the nucleotide sequence is as shown in Fig. 2.

3.  A replicative recombinant plasmid containing a replicative expression vector and a DNA fragment containing a nucleotide sequence capable of encoding the amino acid sequence as shown in Fig. 1.

4.  A transformant containing the recombinant vector as claimed in Claim 3.

5.  A process for preparing a thrombin-binding substance having the amino acid sequence as shown in Fig. 1, which comprises culturing the transformant as claimed in Claim 4 and harvesting a polypeptide produced by the culture.

6.  The process for preparing a thrombin-binding substance according to Claim 5, wherein the polypeptide is glycosylated.

## FIG. 1

AlaProAlaGluProGlnProGlyGlySerGlnCysValGluHisAspCysPheAlaLeu
TyrProGlyProAlaThrPheLeuAsnAlaSerGlnIleCysAspGlyLeuArgGlyHis
LeuMetThrValArgSerSerValAlaAlaAspValIleSerLeuLeuLeuAsnGlyAsp
GlyGlyValGlyArgArgArgLeuTrpIleGlyLeuGlnLeuProProGlyCysGlyAsp
ProLysArgLeuGlyProLeuArgGlyPheGlnTrpValThrGlyAspAsnAsnThrSer
TyrSerArgTrpAlaArgLeuAspLeuAsnGlyAlaProLeuCysGlyProLeuCysVal
AlaValSerAlaAlaGluAlaThrValProSerGluProIleTrpGluGluGlnGlnCys
GluValLysAlaAspGlyPheLeuCysGluPheHisPheProAlaThrCysArgProLeu
AlaValGluProGlyAlaAlaAlaAlaAlaValSerIleThrTyrGlyThrProPheAla
AlaArgGlyAlaAspPheGlnAlaLeuProValGlySerSerAlaAlaValAlaProLeu
GlyLeuGlnLeuMetCysThrAlaProProGlyAlaValGlnGlyHisTrpAlaArgGlu
AlaProGlyAlaTrpAspCysSerValGluAsnGlyGlyCysGluHisAlaCysAsnAla
IleProGlyAlaProArgCysGlnCysProAlaGlyAlaAlaLeuGlnAlaAspGlyArg
SerCysThrAlaSerAlaThrGlnSerCysAsnAspLeuCysGluHisPheCysValPro
AsnProAspGlnProGlySerTyrSerCysMetCysGluThrGlyTyrArgLeuAlaAla
AspGlnHisArgCysGluAspValAspAspCysIleLeuGluProSerProCysProGln
ArgCysValAsnThrGlnGlyGlyPheGluCysHisCysTyrProAsnTyrAspLeuVal
AspGlyGluCysValGluProValAspProCysPheArgAlaAsnCysGluTyrGlnCys
GlnProLeuAsnGlnThrSerTyrLeuCysValCysAlaGluGlyPheAlaProIlePro
HisGluProHisArgCysGlnMetPheCysAsnGlnThrAlaCysProAlaAspCysAsp
ProAsnThrGlnAlaSerCysGluCysProGluGlyTyrIleLeuAspAspGlyPheIle
CysThrAspIleAspGluCysGluAsnGlyGlyPheCysSerGlyValCysHisAsnLeu
ProGlyThrPheGluCysIleCysGlyProAspSerAlaLeuValArgHisIleGlyThr
AspCysAspSerGlyLysValAsp

## FIG. 2

GCACCCGCAGAGCCGCAGCCGGGTGGCAGCCAGTGCGTCGAGCACGACTGCTTCGCGCTC

TACCCGGGCCCCGCGACCTTCCTCAATGCCAGTCAGATCTGCGACGGACTGCGGGGCCAC

CTAATGACAGTGCGCTCCTCGGTGGCTGCCGATGTCATTTCCTTGCTACTGAACGGCGAC

GGCGGCGTTGGCCGCCGGCGCCTCTGGATCGGCCTGCAGCTGCCACCCGGCTGCGGCGAC

CCCAAGCGCCTCGGGCCCCTGCGCGGCTTCCAGTGGGTTACGGGAGACAACAACACCAGC

TATAGCAGGTGGGCACGGCTCGACCTCAATGGGGCTCCCCTCTGCGGCCCGTTGTGCGTC

GCTGTCTCCGCTGCTGAGGCCACTGTGCCCAGCGAGCCGATCTGGGAGGAGCAGCAGTGC

GAAGTGAAGGCCGATGGCTTCCTCTGCGAGTTCCACTTCCCAGCCACCTGCAGGCCACTG

GCTGTGGAGCCCGGCGCCGCGGCTGCCGCCGTCTCGATCACCTACGGCACCCCGTTCGCG

GCCCGCGGAGCGGACTTCCAGGCGCTGCCGGTGGGCAGCTCCGCCGCGGTGGCTCCCCTC

GGCTTACAGCTAATGTGCACCGCGCCGCCCGGAGCGGTCCAGGGGCACTGGGCCAGGGAG

GCGCCGGGCGCTTGGGACTGCAGCGTGGAGAACGGCGGCTGCGAGCACGCGTGCAATGCG

ATCCCTGGGGCTCCCCGCTGCCAGTGCCCAGCCGGCGCCGCCCTGCAGGCAGACGGGCGC

TCCTGCACCGCATCCGCGACGCAGTCCTGCAACGACCTCTGCGAGCACTTCTGCGTTCCC

AACCCCGACCAGCCGGGCTCCTACTCGTGCATGTGCGAGACCGGCTACCGGCTGGCGGCC

GACCAACACCGGTGCGAGGACGTGGATGACTGCATACTGGAGCCCAGTCCGTGTCCGCAG

CGCTGTGTCAACACACAGGGTGGCTTCGAGTGCCACTGCTACCCTAACTACGACCTGGTG

GACGGCGAGTGTGTGGAGCCCGTGGACCCGTGCTTCAGAGCCAACTGCGAGTACCAGTGC

CAGCCCCTGAACCAAACTAGCTACCTCTGCGTCTGCGCCGAGGGCTTCGCGCCCATTCCC

CACGAGCCGCACAGGTGCCAGATGTTTTGCAACCAGACTGCCTGTCCAGCCGACTGCGAC

CCCAACACCCAGGCTAGCTGTGAGTGCCCTGAAGGCTACATCCTGGACGACGGTTTCATC

TGCACGGACATCGACGAGTGCGAAAACGGCGGCTTCTGCTCCGGGGTGTGCCACAACCTC

CCCGGTACCTTCGAGTGCATCTGCGGGCCCGACTCGGCCCTTGTCCGCCACATTGGCACC

GACTGTGACTCCGGCAAGGTGGAC

FIG. 3

```
    10        20        30        40        50        60
CTCGAGCCCTGGCCGATCCGCATGTCAGAGGCTGCCTCGCAGGGGCTGCGCGCAGCGGCA
XhoI

    70        80        90        100       110       120
AGAAGTGTCTGGGCTGGGACGGACAGGAGAGGCTGTCGCCATCGGCGTCCTGTGCCCCTC

    130       140       150       160       170       180
TGCTCCGGCACGGCCCTGTCGCAGTGCCCGCGGCTTTCCCCGGCGCCTGCACGCGGCGCGC

    190       200       210       220       230       240
CTGGGTAACATGCTTGGGGTCCTGGTCCTTGGCGCGCTGGCCCTGGCCGGCCTGGGGTTC
          M  L  G  V  L  V  L  G  A  L  A  L  A  G  L  G  F

    250       260       270       280       290       300
CCCGCACCCGCAGAGCCGCAGCCGGGTGGCAGCCAGTGCGTCGAGCACGACTGCTTCGCG
 P  A  P  A  E  P  Q  P  G  G  S  Q  C  V  E  H  D  C  F  A

    310       320       330       340       350       360
CTCTACCCGGGCCCCGCGACCTTCCTCAATGCCAGTCAGATCTGCGACGGACTGCGGGGC
 L  Y  P  G  P  A  T  F  L  N  A  S  Q  I  C  D  G  L  R  G

    370       380       390       400       410       420
CACCTAATGACAGTGCGCTCCTCGGTGGCTGCCGATGTCATTTCCTTGCTACTGAACGGC
 H  L  M  T  V  R  S  S  V  A  A  D  V  I  S  L  L  L  N  G

    430       440       450       460       470       480
GACGGCGGCGTTGGCCGCCGGCGCCTCTGGATCGGCCTGCAGCTGCCACCCGGCTGCGGC
 D  G  G  V  G  R  R  R  L  W  I  G  L  Q  L  P  P  G  C  G

    490       500       510       520       530       540
GACCCCAAGCGCCTCGGGCCCCTGCGCGGCTTCCAGTGGGTTACGGGAGACAACAACACC
 D  P  K  R  L  G  P  L  R  G  F  Q  W  V  T  G  D  N  N  T

    550       560       570       580       590       600
AGCTATAGCAGGTGGGCACGGCTCGACCTCAATGGGGCTCCCCTCTGCGGCCCGTTGTGC
 S  Y  S  R  W  A  R  L  D  L  N  G  A  P  L  C  G  P  L  C

    610       620       630       640       650       660
GTCGCTGTCTCCGCTGCTGAGGCCACTGTGCCCAGCGAGCCGATCTGGGAGGAGCAGCAG
 V  A  V  S  A  A  E  A  T  V  P  S  E  P  I  W  E  E  Q  Q

    670       680       690       700       710       720
TGCGAAGTGAAGGCCGATGGCTTCCTCTGCGAGTTCCACTTCCCAGCCACCTGCAGGCCA
 C  E  V  K  A  D  G  F  L  C  E  F  H  F  P  A  T  C  R  P

    730       740       750       760       770       780
CTGGCTGTGGAGCCCGGCGCCGCGGCTGCCGCCGTGTCTCGATCACCTACGGCACCCCGTTC
 L  A  V  E  P  G  A  A  A  A  V  S  I  T  Y  G  T  P  F

    790       800       810       820       830       840
GCGGCCCGCGGAGCGGACTTCCAGGCGCTGCCGGTGGGCAGCTCCGCCGCGGTGGCTCCC
 A  A  R  G  A  D  F  Q  A  L  P  V  G  S  S  A  A  V  A  P

    850       860       870       880       890       900
CTCGGCTTACAGCTAATGTGCACCGCGCCGCCGGAGCGGTCCAGGGGCACTGGGCCAGG
 L  G  L  Q  L  M  C  T  A  P  P  G  A  V  Q  G  H  W  A  R

    910       920       930       940       950       960
GAGGCGCGGGCGCTTGGGACTGCAGCGTGGAGAACGGCGGCTGCGAGCACGCGTGCAAT
 E  A  P  G  A  W  D  C  S  V  E  N  G  G  C  E  H  A  C  N

    970       980       990       1000      1010      1020
GCGATCCCTGGGGCTCCCCGCTGCCAGTGCCCAGCCGGCGCCGCCCTGCAGGCAGACGGG
 A  I  P  G  A  P  R  C  Q  C  P  A  G  A  A  L  Q  A  D  G

    1030      1040      1050      1060      1070      1080
CGCTCCTGCACCGCATCCGCGACGCAGTCCTGCAACGACCTCTGCGAGCACTTCTGCGTT
 R  S  C  T  A  S  A  T  Q  S  C  N  D  L  C  E  H  F  C  V

    1090      1100      1110      1120      1130      1140
CCCAACCCCGACCAGCCGGGCTCCTACTCGTGCATGTGCGAGACCGGCTACCGGCTGGCG
 P  N  P  D  Q  P  G  S  Y  S  C  M  C  E  T  G  Y  R  L  A

    1150      1160      1170      1180      1190      1200
GCCGACCAACACCGGTGCGAGGACGTGGATGACTGCATACTGGAGCCCAGTCCGTGTCCG
 A  D  Q  H  R  C  E  D  V  D  D  C  I  L  E  P  S  P  C  P

    1210      1220      1230      1240      1250      1260
CAGCGCTGTGTCAACACACAGGGGTGGCTTCGAGTGCCACTGCTACCCTAACTACGACCTG
 Q  R  C  V  N  T  Q  G  G  F  E  C  H  C  Y  P  N  Y  D  L

    1270      1280      1290      1300      1310      1320
GTGGACGGCGAGTGTGTGGAGCCCGTGGACCCGTGCTTCAGAGCCAACTGCGAGTACCAG
 V  D  G  E  C  V  E  P  V  D  P  C  F  R  A  N  C  E  Y  Q

    1330      1340      1350      1360      1370      1380
TGCCAGCCCCTGAACCAAACTAGCTACCTCTGCGTCTGCGCGCCGAGGGCTTCGCGCCCATT
 C  Q  P  L  N  Q  T  S  Y  L  C  V  C  A  E  G  F  A  P  I

    1390      1400      1410      1420      1430      1440
CCCCACGAGCCGCACAGGTGCCAGATGTTTTGCAACCAGACTGCCTGTCCAGCCGACTGC
 P  H  E  P  H  R  C  Q  M  F  C  N  Q  T  A  C  P  A  D  C

    1450      1460      1470      1480      1490      1500
GACCCCAACACCCAGGCTAGCTGTGAGTGCCCTGAAGGCTACATCCTGGACGACGGTTTC
 D  P  N  T  Q  A  S  C  E  C  P  E  G  Y  I  L  D  D  G  F

    1510      1520      1530      1540      1550      1560
ATCTGCACGGACATCGACGAGTGCGAAAAACGGCGGCTTCTGCTCCGGGGTGTGCCACAAC
 I  C  T  D  I  D  E  C  E  N  G  G  F  C  S  G  V  C  H  N

    1570      1580      1590      1600      1610      1620
CTCCCCGGTACCTTCGAGTGCATCTGCGGGCCCGACTCGGCCCTTGTCCGCCACATTGGC
 L  P  G  T  F  E  C  I  C  G  P  D  S  A  L  V  R  H  I  G

    1630      1640
ACCGACTGTGACTCCGGCAAGGTGGACTGACTCGAG
 T  D  C  D  S  G  K  V  D  *  XhoI
```

FIG. 4

EP 0 445 681 A2